(19) Europäisches Patentamt
European Patent Office
Office européen des brevets

(11) Publication number: **0 401 705 A2**

(12) **EUROPEAN PATENT APPLICATION**

(21) Application number: **90110475.2**

(22) Date of filing: **01.06.90**

(51) Int. Cl.$^{5}$: **A61K 31/66, A61K 31/365, A61K 31/235, A61K 31/35, A61K 31/415, A61K 31/015, A61K 37/22, A61K 31/00, A61K 31/22**

(30) Priority: **05.06.89 US 361520**

(43) Date of publication of application: **12.12.90 Bulletin 90/50**

(84) Designated Contracting States: **AT BE CH DE DK ES FR GB GR IT LI LU NL SE**

(71) Applicant: **E.R. Squibb & Sons, Inc.**
**Lawrenceville-Princeton Road**
**Princeton, N.J. 08540-4000(US)**

(72) Inventor: **Eisman, Martin**
**10 West Shore Drive**
**Pennington N.J.(US)**

(74) Representative: **Vossius & Partner**
**Siebertstrasse 4 P.O. Box 86 07 67**
**D-8000 München 86(DE)**

(54) **Use of an HMG CoA reductase inhibitor and/or a squalene synthetase inhibitor for treating peripheral atherosclerotic disease.**

(57) A use of a pharmaceutical composition is provided for treating peripheral atherosclerotic disease (arteriosclerosis obliterans) and/or intermittent claudication containing an HMG CoA reductase inhibitor alone and/or inhibitor of the enzyme squalene synthetase and optionally a pharmaceutical which reduces serum cholesterol by a mechanism other than inhibiting production of the enzyme HMG CoA reductase or the enzyme squalene synthetase, for example, probucol or gemfibrozil.

EP 0 401 705 A2

# USE OF AN HMG CoA REDUCTASE INHIBITOR AND/OR A SQUALENE SYNTHETASE INHIBITOR FOR TREATING PERIPHERAL ATHEROSCLEROTIC DISEASE

The present invention relates to the use of an inhibitor of 3-hydroxy-3-methylglutaryl coenzyme A (HMG CoA) reductase or an inhibitor of squalene synthetase for treating peripheral atherosclerotic disease (arteriosclerosis obliterans).

Harrison's Principles of Internal Medicine, 11th Edition, 1987, Editors-E. Braunwald et al, Chapter 198, pp 1040-1046 by D. Eugene Strandness, Jr., discusses vascular diseases of the extremities - also referred to as peripheral vascular disorders (Merck Manual, 15th Ed, Chapter 30, pp 555-557) - which include arteriosclerosis obliterans. "Symptoms and signs that develop secondary to arteriosclerosis obliterans rarely have an abrupt onset, since the process is a gradual, progressive one. The most common symptom occurs with exercise and is termed intermittent claudication, i.e., the pain that occurs in a muscle(s) with an inadequate blood supply that is stressed by exercise (page 1041)."

There are several different classes of compounds which have serum cholesterol lowering properties. Some of these compounds are inhibitors of the enzyme HMG CoA reductase which is essential in the production of cholesterol, such as mevastatin (disclosed in U. S. Patent No. 3,983,140), lovastatin also referred to as mevinolin (disclosed in U. S. Patent No. 4,231,938), pravastatin (disclosed in U. S. Patent No. 4,346,227) and velostatin also referred to as synvinolin (disclosed in U. S. Patents Nos. 4,448,784 and 4,450,171).

Other compounds which lower serum cholesterol may do so by an entirely different mechanism than the HMG CoA reductase inhibitors. For example, serum cholesterol may be lowered through the use of bile acid sequestrants such as cholestyramine, colestipol, DEAE-Sephadex and poly(diallylmethylamine) derivatives (such as disclosed in U. S. Patents Nos. 4,759,923 and 4,027,009) or through the use of antihyperlipoproteinemics such as probucol and gemfibrozil which apparently lower serum "low density lipoproteins" (LDL) and/or converts LDL into high density lipoproteins (HDL).

U. S. Patent No. 4,759,923 mentioned above discloses that poly(diallylmethylamine) derivatives which are bile salt sequestrants may be used in conjunction with drugs which reduce serum cholesterol by mechanisms other than sequestration, such as clofibrate, nicotinic acid, probucol, neomycin, p-aminosalicylic acid or mevinolin (also referred to as lovastatin).

Squalene synthetase is a microsomal enzyme which catalyzes the reductive dimerization of two molecules of farnesyl pyrophosphate (FPP) in the presence of nicotinamide adenine dinucleotide phosphate (reduced form) (NADPH) to form squalene (Poulter, C. D.; Rilling, H. C., in "Biosynthesis of Isoprenoid Compounds", Vol. I, Chapter 8, pp. 413-441, J. Wiley and Sons, 1981 and references therein). This enzyme is the first committed step of the de novo cholesterol biosynthetic pathway. The selective inhibition of this step should allow the essential pathways to isopentenyl tRNA, ubiquinone, and dolichol to proceed unimpeded. Squalene synthetase, along with HMG-CoA reductase has been shown to be down-regulated by receptor mediated LDL uptake (Faust, J. R.; Goldstein, J. L.; Brown, M. S. Proc. Nat. Acad. Sci. USA, 1979, 76, 5018-5022), lending credence to the proposal that inhibiting squalene synthetase will lead to an up-regulation of LDL receptor levels, as has been demonstrated for HMG-CoA reductase, and thus ultimately should be useful for the treatment and prevention of hypercholesterolemia and atherosclerosis.

One approach to inhibitors of squalene synthetase is to design analogs of the substrate FPP. It is clear from the literature that the pyrophosphate moiety is essential for binding to the enzyme. However, such pyrophosphates are unsuitable as components of pharmacological agents due to their chemical and enzymatic lability towards allylic C-O cleavage, as well as their susceptibility to metabolism by phosphatases.

P. Ortiz de Montellano et al in J. Med. Chem., 1977, 20, 243-249 describe the preparation of a series of substituted terpenoid pyrophosphates (Table A), and have shown these to be competitive inhibitors of the squalene synthetase enzyme. These substances retain the unstable allylic pyrophosphate moiety of FPP.

Table A

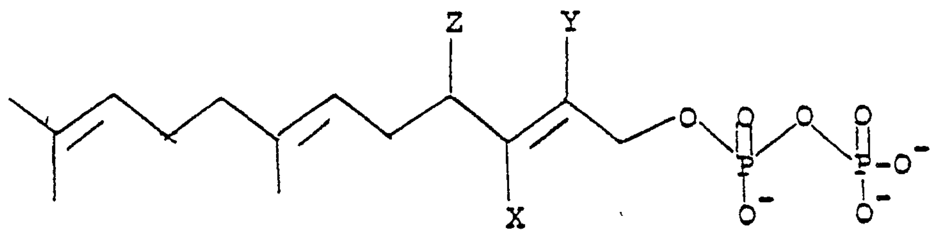

| No. | X | Y | Z |
|---|---|---|---|
| 1 | $CH_3$ | $CH_3$ | H |
| 2 | H | H | H |
| 3 | $C_2H_5$ | H | H |
| 4 | I | H | H |
| 5 | H | I | H |
| 6 | $CH_3$ | H | $SCH_3$ |

Corey and Volante, J. Am. Chem. Soc. 1976, 98, 1291-3, have prepared FPP analog A and presqualene pyrophosphate (PSQ-PP) analog B as inhibitors of squalene biosynthesis. (Presqualene pyrophosphate is an intermediate in the conversion of FPP to squalene). These inhibitors possess methylene groups in place of the allylic oxygen moiety of FPP and PSQ-PP, but still retain the chemically and enzymatically unstable pyrophosphate linkage.

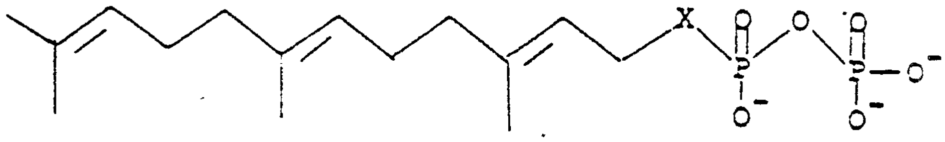

A $X = CH_2$

FPP $X = O$

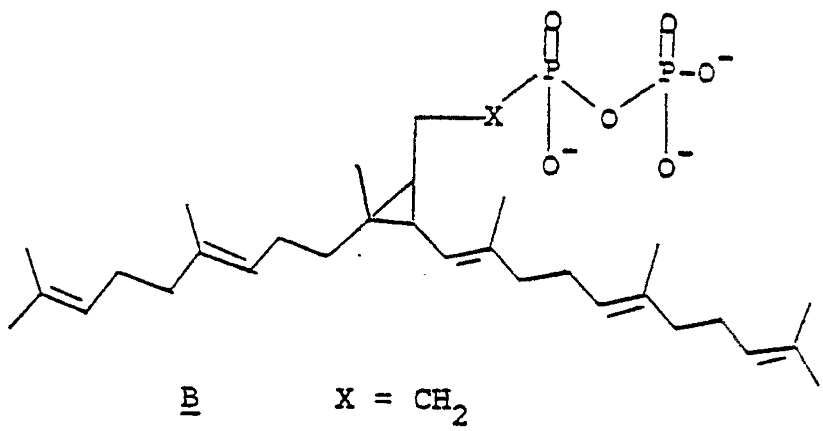

B $X = CH_2$

PSQ-PP $X = O$

Poulter and co-workers have prepared cyclopropane C (Sandifer, R. M., et al., J. Am. Chem. Soc. 1982, 104, 7376-8) which in the presence of inorganic pyrophosphate is an intermediate analog inhibitor of the enzyme squalene synthetase.

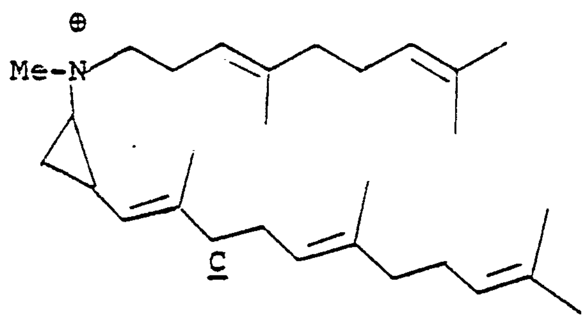

C

Altman and co-workers, Bertolino, A., et al., Biochim. Biophys. Acta. 1978, 530, 17-23, reported that farnesyl amine and related derivatives D inhibit squalene synthetase, but provide evidence that this inhibition is non-specific and probably related to membrane disruption.

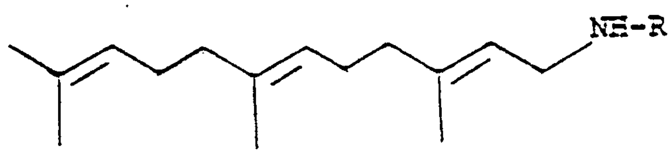

R = H, $CH_2CH_2OH$, $CH_2CH_2OCH_3$

D

Poulter, C.D., et al, J. Org. Chem., 1986, 51, 4768, prepared compound E in a demonstration of a synthetic method, but did not report any biological data.

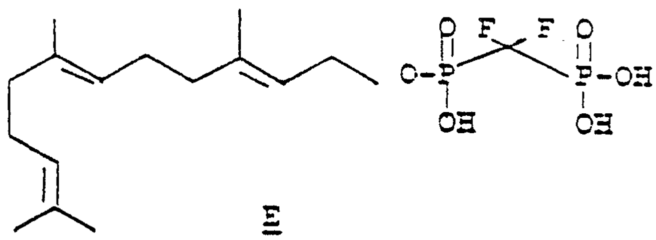

E

Poulter, C.D., Stremler, K.E., J.A.C.S., 1987, 109, 5542 describes the synthesis and biological evaluation of compounds having structure F. These compounds were evaluated as alternative substrates for avian liver farnesyl diphosphate and lemon peel cyclase.

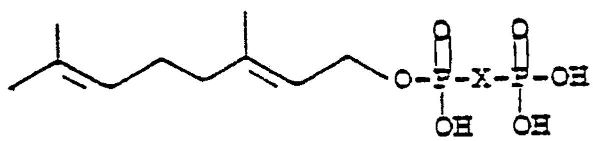

F X=$CH_2$, $CF_2$

McClard, R. W. and Poulter, C.D., et al., J.A.C.S. 1987, 109, 5544, reported that phosphinylphosphonates G and H were competitive inhibitors of the 1'-4-condensation between isopentenyl diphosphate and geranyl diphosphate catalyzed by avian liver farnesyl diphosphate synthetase. Phosphinylphosphonates G and H had Ki's of 19μM and 71μM, respectively. They also reported the speculative isolation of the farnesyl phosphinylphosphonate I, and the geranyl phosphinylphosphonate J from the enzymatic reaction of G with geranyl pyrophosphate or dimethylallyl pyrophosphate, respectively. The structures of I and J were tentatively assigned based on relative TLC mobilities. They hypothesized that I could be a potential inhibitor of squalene synthetase.

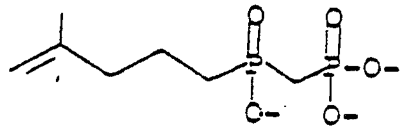

G

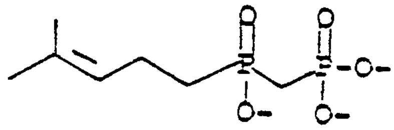

H

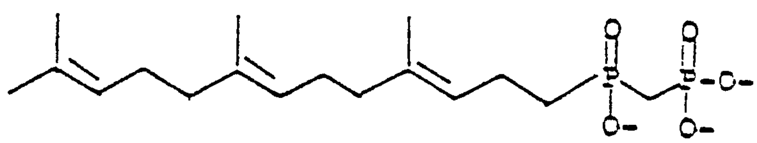

I

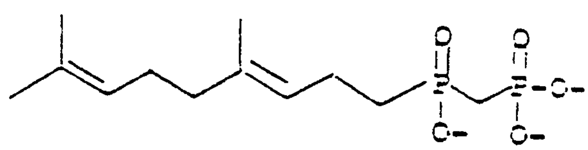

J

Capson, T.L., PhD dissertation, June 1987, Dept. of Medicinal Chemistry, the University of Utah, Abstract, Table of Contents, pp. 16, 17, 40-43, 48-51, Summary, discloses cyclopropanes of the structure discloses cyclopropanes of the structure

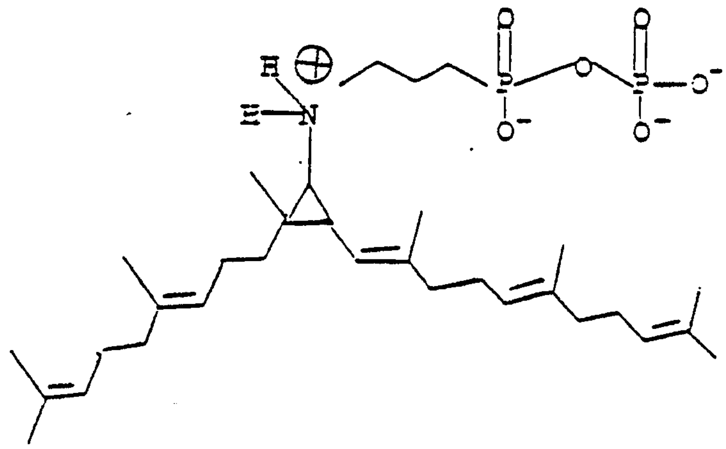

as intermediate analog inhibitors of squalene synthetase.

S. A. Biller et al., Journal of Medicinal Chemistry, 1988, Vol. 31, No. 10, pp 1869 to 1871 disclose that isoprenoid (phosphinylmethyl) phosphonates (PMPs) inhibit squalene synthetase. These phosphonates have the structures

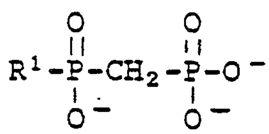

2a-d

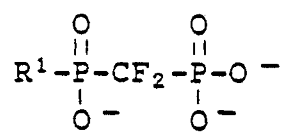

3a,b

$R^1$

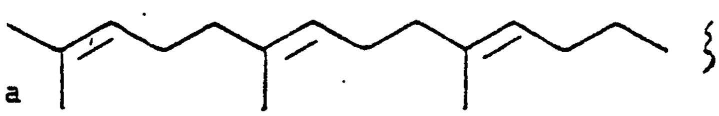

a

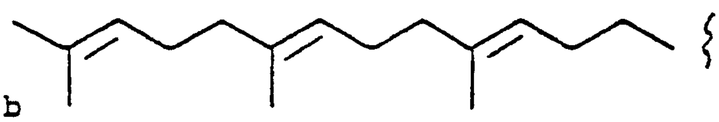

b

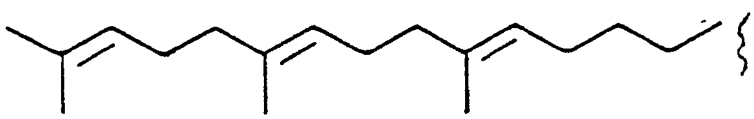

c

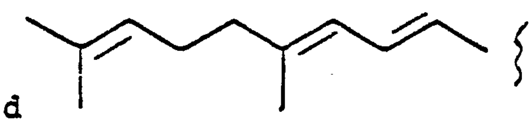

d

In accordance with the present invention, the use of a pharmaceutical composition is provided for

treating peripheral atherosclerotic disease,also referred to as arteriosclerosis obliterans, and for treating intermittent claudication, containing a cholesterol lowering drug which is an inhibitor of the enzyme 3-hydroxy-3-methylglutaryl coenzyme A (HMG CoA) reductase or an inhibitor of the enzyme squalene synthetase, a combination thereof or a combination of each with another pharmaceutical for lowering cholesterol, wherein a therapeutically effective amount of the above type compounds is systemically, such as orally or parenterally, administered over a prolonged period.

Where the combination of the HMG CoA reductase inhibitor and squalene synthetase inhibitor are employed, the HMG CoA reductase inhibitor will be employed in a weight ratio to the squalene synthetase inhibitor of within the range of from about 0.001:1 to about 1000:1 and preferably from about 0.05:1 to about 100:1.

The pharmaceutical (also referred to as other serum cholesterol lowering agent) reduces serum cholesterol and/or inhibits cholesterol biosynthesis by a mechanism other than by inhibiting production of the enzyme squalene synthetase or 3-hydroxy-3-methyl-glutaryl coenzyme A (HMG CoA) reductase, such as a bile salt sequestrant or antihyperlipoproteinemic agent which inhibits formation of LDL or converts LDL to HDL. The HMG CoA reductase inhibitor or squalene synthetase inhibitor will be employed in a weight ratio to the "pharmaceutical" of within the range of from about 0.001:1 to about 1000:1 and preferably from about 0.05:1 to about 100:1.

Cholesterol lowering drugs or drugs which are inhibitors of cholesterol biosynthesis which may be used in the method of the invention include HMG CoA reductase inhibitors or squalene synthetase inhibitors.

The HMG CoA reductase inhibitors suitable for use herein include, but are not limited to, mevastatin and related compounds as disclosed in U. S. Patent No. 3,983,140, lovastatin (mevinolin) and related compounds as disclosed in U. S. Patent No. 4,231,938, pravastatin and related compounds such as disclosed in U. S. Patent No. 4,346,227, velostatin (synvinolin) and related compounds as disclosed in U. S. Patents Nos. 4,448,784 and 4,450,171, with lovastatin, pravastatin or velostatin being preferred. Other HMG CoA reductase inhibitors which may be employed herein include, but are not limited to, fluindostatin (Sandoz XU-62-320), pyrazole analogs of mevalonolactone derivatives as disclosed in U. S. Patent No. 4,613,610, indene analogs of mevalonolactone derivatives as disclosed in PCT application WO 86/03488, 6-[2-(substituted-pyrrol-1-yl)alkyl]pyran-2-ones and derivatives thereof as disclosed in U. S. Patent No. 4,647,576, Searle's SC-45355 (a 3-substituted pentanedioic acid derivative) dichloroacetate, imidazole analogs of mevalonolactone as disclosed in PCT application WO 86/07054, 3-carboxy-2-hydroxy-propane-phosphonic acid derivatives as disclosed in French Patent No. 2,596,393, 2,3-di-substituted pyrrole, furan and thiophene derivatives as disclosed in European Patent Application No. 0221025, naphthyl analogs of mevalonolactone as disclosed in U. S. Patent No. 4,686,237, octahydro-naphthalenes such as disclosed in U. S. Patent No. 4,499,289, keto analogs of mevinolin (lovastatin) as disclosed in European Patent Application No. 0,142,146 A2, as well as other known HMG CoA reductase inhibitors.

In addition, compounds useful in inhibiting HMG CoA reductase suitable for use herein are disclosed in GB 2205837 which compounds have the moiety

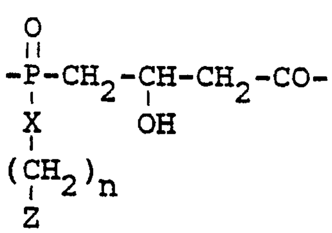

wherein X is -O- or -NH-, n is 1 or 2 and Z is a hydrophobic anchor.

Examples of such compounds include (S)-4-[[[4'-fluoro-3,3',5-trimethyl[1,1'-biphenyl]-2-yl]methoxy]-methoxyphosphinyl]-3-hydroxy-butanoic acid, methyl ester or its monolithium salt,
(S)-4-[[[4'-fluoro-3,3',5-trimethyl[1,1'-biphenyl]-2-yl]methoxy]hydroxyphosphinyl]-3-hydroxybutanoic acid, dilithium salt,
(3S)-4-[[[4'-fluoro-3,3',5-trimethyl[1,1'-biphenyl]-2-yl]methoxy]methylphosphinyl]-3-hydroxybutanoic acid, monolithium salt,
(S)-4-[[[2,4-dichloro-6-[(4-fluorophenyl)methoxy]phenyl]methoxy]methoxyphosphinyl]-3-hydroxybutanoic acid, monolithium salt,
(3S)-4-[[[2,4-dichloro-6-[(4-fluorophenyl)methoxy]phenyl]methoxy]hydroxyphosphinyl]-3-hydroxybutanoic acid, dilithium salt,

(3S)-4-[[2,4-dichloro-6-[(4-fluorophenyl)methoxy]phenyl]methoxy]methylphosphinyl]-3-hydroxybutanoic acid, or its methyl ester, and
(S)-4-[[[[4′-fluoro-3,3′,5-trimethyl[1,1′-biphenyl-2-yl]methyl]amino]methoxyphosphinyl]-3-hydroxybutanoic aicd, monolithium salt.

Another class of HMG CoA reductase inhibitors suitable for use herein include compounds disclosed in GB 2205838, which compounds have the moiety

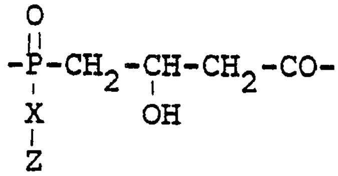

wherein X is $-CH_2-$ $-CH_2-CH_2-$, $-CH=CH-$, $-CH_2CH_2CH_2-$, $-C\equiv C-$ or $-CH_2O-$, where O is linked to Z, and Z is a hydrophobic anchor.

Examples of such compounds include (S)-4-[[(E)-2-[4′-fluoro-3,3′,5-trimethyl[1,1′-biphenyl]-2-yl]ethenyl]-hydroxyphosphinyl]-3-hydroxybutanoic acid or its dilithium salt;
(S)-4-[[2-[4′-fluoro-3,3′,5-trimethyl-[1,1′-biphenyl]-2-yl]ethyl]hydroxyphosphinyl]-3-hydroxybutanoic acid, methyl ester or mono- or di-alkali metal salts thereof;
(S)-4-[[[4′-fluoro-3,3′,5-trimethyl-[1,1′-biphenyl]-2-yl]ethynyl]methoxyphosphinyl]-3-hydroxybutanoic acid or the methyl ester thereof;
(5Z)-4-[[2-[4′-fluoro-3,3′,5-trimethyl-[1,1′-biphenyl]-2-yl]ethenyl]hydroxyphosphinyl]-3-hydroxybutanoic acid, methyl esters thereof;
(S)-4-[[2-[3-(4-fluorophenyl)-1-(1-methylethyl)-1H-indol-2-yl]ethyl]methoxyphosphinyl]-3-hydroxybutanoic acid, methyl esters;
(S)-4-[[2-[[1,1′-biphenyl]-2-yl]ethyl]methoxyphosphinyl-3-hydroxybutanoic acid, methyl ester;
(S)-4-[[2-[4′-fluoro-3,3′,5-trimethyl-[1,1′-biphenyl]-2-yl]ethyl]hydroxyphosphinyl]-3-hydroxybutanoic acid, dilithium salt;
(S)-4-[[-2-[4′-fluoro-3,3′,5-trimethyl-[1,1′-biphenyl]-2-yl]ethynyl]hydroxyphosphinyl]-3-hydroxybutanoic acid, dilithium salt;
(SZ)-4-[[2-[4′-fluoro-3,3′,5-trimethyl-[1,1′-biphenyl]-2-yl]ethenyl]hydroxyphosphinyl]-3-hydroxybutanoic acid, dilithium salt;
(S)-4-[[2-[3-(4-fluorophenyl)-1-(1-methylethyl)-1H-indol-2-yl]ethyl]hydroxyphosphinyl]3-hydroxybutanoic acid, dilithium salt;
(S)-4-[[2-[(1,1′-biphenyl]-2-yl]ethyl]hydroxyphosphinyl]-3-butanoic acid, dilithium salt;
(S)-4-(hydroxymethoxyphosphinyl)-3-[[(1,1-dimethylethyl)diphenylsilyl]oxy]butanoic acid, methyl ester, or its dicyclohexylamine (1:1) salt;
(S)-4-[[2-[1-(4-fluorophenyl)-3-(1-methylethyl)-1-indol-2-yl]ethynyl]hydroxyphosphinyl]-3-hydroxybutanoic acid or its dilithium salt or methyl ester thereof;
(S)-4-[[2-[1-(4-fluorophenyl)-3-(1-methylethyl)-1H-indol-2-yl]ethyl]hydroxyphosphinyl]-3-hydroxybutanoic acid or its dilithium salt or methyl ester thereof;
(E)-4-[[2-[3-(4-fluorophenyl)-1-(1-methylethyl)-1H-indol-2-yl]ethenyl]hydroxyphosphinyl]-3-hydroxybutanoic acid or its dilithium salt or methyl ester thereof;
4-[[2-[4′-fluoro-3,3′,5-trimethyl[1,1′-biphenyl]-2-yl]ethyl]hydroxyphosphinyl]-3-hydroxy-butanoic acid or its dilithium salt or methyl ester thereof;
(E)-4-[[2-[4′-fluoro-3,3′,5-trimethyl[1,1′-biphenyl]-2-yl]ethenyl]hydroxyphosphinyl]-3-hydroxybutanoic acid or its dilithium salt or methyl ester thereof;
(S)-4-[[[2,4-dimethyl-6-[(4-fluorophenyl)methoxy]phenyl]ethyl]hydroxyphosphinyl]-3-hydroxybutanoic acid or its dilithium salt or methyl ester thereof;
(S)-4-[[[2,4-dimethyl-6-[(4-fluorophenyl)methoxy]phenyl]ethynyl]hydroxyphosphinyl]-3-hydroxybutanoic acid or its dilithium salt or methyl ester thereof;
(S)-4-[[2-[3,5-dimethyl[1,1′-biphenyl)-2-yl]ethyl)hydroxyphosphinyl]-3-hydroxybutanoic acid or its dilithium salt or methyl ester thereof;
(S)-4-[[2-[4′-fluoro-3,5-dimethyl[1,1′-biphenyl]-2-yl]ethyl]hydroxyphosphinyl]-3-hydroxybutanoic acid or its dilithium salt or methyl ester thereof;
(S)-4-[[2-[[1,1′-biphenyl]-2-yl]ethynyl]hydroxyphosphinyl]-3-hydroxybutanoic acid or its dilithium salt or methyl ester thereof;

(S)-4-[[2-(5-(4-fluorophenyl)-3-(1-methylethyl)-1-phenyl-1H-pyrazol-4-yl]ethynyl]methoxyphosphinyl]-3-hydroxybutanoic acid, methyl ester;

(S)-4-[[2-[5-(4-fluorophenyl)-3-(1-methylethyl)-1-phenyl-1H-pyrazol-4-yl]ethynyl]hydroxyphosphinyl]-3-hydroxybutanoic acid, dilithium salt;

(E)-4-[[2-[5-(4-fluorophenyl)-3-(1-methylethyl)-1-phenyl-1H-pyrazol-4-yl]ethenyl]methoxyphosphinyl]-3-hydroxybutanoic acid, methyl ester;

(E)-4-[[2-[5-(4-fluorophenyl)-3-(1-methylethyl)-1-phenyl-1H-pyrazol-4-yl]ethenyl]hydroxyphosphinyl]-3-hydroxybutanoic acid, dilithium salt;

(S)-4-[[2-[5-(4-fluorophenyl)-3-(1-methylethyl)-1-phenyl-1H-pyrazol-4-yl]ethyl]methoxyphosphinyl]-3-hydroxybutanoic acid, methyl ester;

(S)-4-[[2-[5-(4-fluorophenyl)-3-(1-methylethyl)-1-phenyl-1H-pyrazol-4-yl]ethyl]hydroxyphosphinyl]-3-hydroxybutanoic acid, dilithium salt;

(S)-4-[[2-[3-(4-fluorophenyl)-5-(1-methylethyl)-1-phenyl-1H-pyrazol-4-yl]ethyl]methoxyphosphinyl]-3-hydroxybutanoic acid, methyl ester;

(S)-4-[[2-[3-(4-fluorophenyl)-5-(1-methylethyl)-1-phenyl-1H-pyrazol-4-yl]ethyl]hydroxyphosphinyl]-3-hydroxybutanoic acid, dilithium salt;

(S)-4-[[2-[3-(4-fluorophenyl)-5-(1-methylethyl)-1-phenyl-1H-pyrazol-4-yl]ethynyl]methoxyphosphinyl]-3-hydroxybutanoic acid, methyl ester;

(S)-4-[[2-[3-(4-fluorophenyl)-5-(1-methylethyl)-1-phenyl-1H-pyrazol-4-yl]ethynyl]hydroxyphosphinyl]-3-hydroxybutanoic acid, dilithium salt;

(S)-4-[[[4-(4-fluorophenyl)-1-(1-methylethyl)-3-phenyl-1H-pyrazol-5-yl]ethynyl]methoxyphosphinyl]-3-hydroxybutanoic acid, methyl ester;

(S)-4-[[[4-(4-fluorophenyl)-1-(1-methylethyl)-3-phenyl-1H-pyrazol-5-yl]ethynyl]hydroxyphosphinyl]-3-hydroxybutanoic acid, dilithium salt;

(S)-4-[[2-[4-(4-fluorophenyl)-1-(1-methylethyl)-3-phenyl-1H-pyrazol-5-yl]ethyl]methoxyphosphinyl]-3-hydroxybutanoic acid, methyl ester;

(S)-4-[[2-[4-(4-fluorophenyl)-1-(1-methylethyl)-3-phenyl-1H-pyrazol-5-yl]ethyl]hydroxyphosphinyl]-3-hydroxybutanoic acid, dilithium salt;

(S)-4-[[[1-(4-fluorophenyl)-4-(1-methylethyl)-2-phenyl-1H-imidazole-5-yl]ethynyl]methox-phosphinyl]-3-hydroxybutanoic acid, methyl ester;

(S)-4-[[[1-(4-fluorophenyl)-4-(1-methylethyl)-2-phenyl-1H-imidazol-5-yl]ethynyl]methoxyphosphinyl]-3-hydroxybutanoic acid, methyl ester;

(S)-4-[[2-[1-(4-fluorophenyl)-4-(1-methylethyl)-2-phenyl-1H-imidazol-5-yl]ethyl]methoxyphosphinyl]-3-hydroxybutanoic acid, methyl ester;

(S)-4-[[2-[1-(4-fluorophenyl)-4-(1-methylethyl)-2-phenyl-1H-imidazol-5-yl]ethyl]hydroxyphosphinyl]-3-hydroxybutanoic acid, dilithium salt;

(S)-4-[[[2-(cyclohexylmethyl)-4,6-dimethylphenyl]ethynyl]hydroxyphosphinyl]-3-hydroxybutanoic acid or its dilithium salt or methyl ester thereof;

4-[[2-[2-(cyclohexylmethyl)-4,6-dimethylphenyl]ethenyl]hydroxyphosphinyl]-3-hydroxybutanoic acid or its dilithium salt or methyl ester thereof;

(S)-4-[[2-[2-(cyclohexylmethyl)-4,6-dimethylphenyl]ethyl]hydroxyphosphinyl]-3-hydroxybutanoic acid or its dilithium salt or methyl ester thereof;

4-[[[[4'-fluoro-3,3',5-trimethyl[1,1'-biphenyl]-2-yl]oxy]methyl]hydroxyphosphinyl]-3-hydroxybutanoic acid or its dilithium salt or methyl ester thereof;

4-[[[4'-fluoro-3,3',5-trimethyl[1,1'-biphenyl]-2-yl]methyl]hydroxyphosphinyl]-3-hydroxybutanoic acid or its dilithium salt or methyl ester thereof;

(S)-4-[[[1-(4-fluorophenyl)-3-methyl-2-naphthalenyl]ethynyl]hydroxyphosphinyl]-3-hydroxybutanoic acid or its dilithium salt or methyl ester thereof;

(E)-4-[[2-[1-(4-fluorophenyl)-3-methyl-2-naphthalenyl]ethenyl]hydroxyphosphinyl]-3-hydroxybutanoic acid or its dilithium salt or methyl ester thereof; (S)-4-[[2-[1-(4-fluorophenyl)-3-methyl-2-naphthalenyl]ethyl]-hydroxyphosphinyl]-3-hydroxybutanoic acid or its dilithium salt or methyl ester thereof;

4-[[3-[4'-fluoro-3,3',5-trimethyl[1,1'-biphenyl]-2-yl]propyl]methoxyphosphinyl]-3-hydroxybutanoic acid, methyl ester;

4-[[3-[4'-fluoro-3,3',5-trimethyl[1,1'-biphenyl]-2-yl]propyl]hydroxyphosphinyl]-3-hydroxybutanoic acid, dilithium salt;

[1S-[1<a(R*),2<a,4a<b,8<b,8a<a]]-4-[[2-[8-(2,2-dimethyl-1-oxobutoxy)decahydro-2-methyl-1-naphthalenyl]-ethyl]methoxyphosphinyl]-3-hydroxybutanoic acid, methyl ester;

[1S-[1<a(R*),2<a,4a<b,8<b,8a<a]]-4-[[2-[8-(2,2-dimethyl-1-oxobutoxy)decahydro-2-methyl-1-naphthalenyl]-

ethyl]hydroxyphosphinyl]-3-hydroxybutanoic acid, dilithium salt;
(S)-4-[[[3′-(4-fluorophenyl)spiro]cyclopentane-1,1′-[1H]indene]-2-yl]ethynyl]methoxyphosphinyl]-3-hydroxybutanoic acid, methyl ester; and
(S)-4-[[[3′-(4-fluorophenyl)spiro]cyclopentane-1,1′-[1H]indene]-2-yl]ethynyl]hydroxyphosphinyl]-3-hydroxybutanoic acid, dilithium salt.

The squalene synthetase inhibitors suitable for use herein include, but are not limited to, those disclosed by Biller et al., supra, including isoprenoid (phosphinylmethyl)phosphonates such as those of the formula

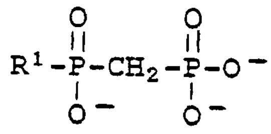

I

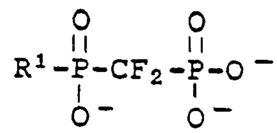

II

$R^1$

a 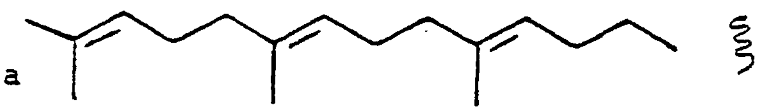

b 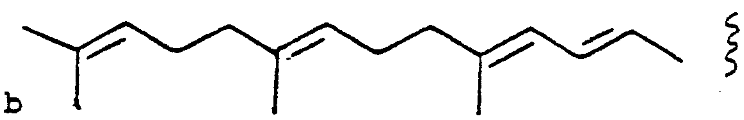

c 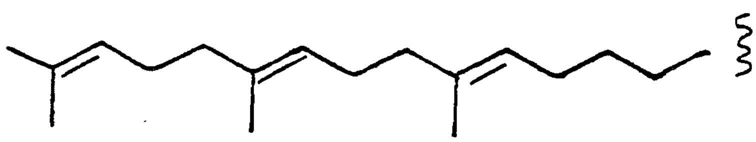

d 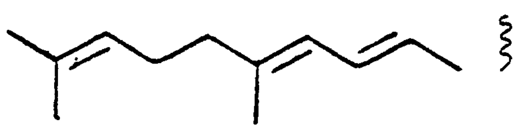

including the triacids thereof, triesters thereof and tripotassium and trisodium salts thereof as well as other squalene synthetase inhibitors disclosed in pending U.S. patent application Serial No. 141,744, filed January 11, 1988 and in Biller et al, J. Med. Chem., 1988, Vol. 31, No. 10, pp 1869 to 1871.

In addition, other squalene synthetase inhibitors suitable for use herein include the terpenoid pyrophosphates disclosed by P. Ortiz de Montellano et al., J. Med. Chem.; 1977, 20, 243-249, the farnesyl diphosphate analog A and presqualene pyrophosphate (PSQ-PP) analogs as disclosed by Corey and Volante, J. Am. Chem. Soc. 1976, 98, 1291-1293, phosphinylphosphonates reported by McClard, R. W. et al., J.A.C.S., 1987, 109, 5544 and cyclopropanes reported by Capson, T.L., PhD dissertation, June, 1987, Dept. Med. Chem. U. of Utah, Abstract, Table of Contents, pp. 16, 17, 40-43, 48-51, Summary.

The disclosure of the above-mentioned patents and patent applications are incorporated herein by reference.

Preferred are pravastatin, lovastatin or velostatin or a squalene synthetase inhibitor such as disclosed by Biller et al., supra or combinations thereof which include a weight ratio of the HMG CoA reductase inhibitor:squalene synthetase inhibitor of from about 0.05:1 to about 100:1.

The "pharmaceutical" or other serum cholesterol lowering agents which function other than by inhibiting the enzyme HMG CoA reductase or squalene synthetase suitable for use herein include, but are not limited to, antihyperlipoproteinemic agents such as probucol, and gemfibrozil and related compounds as disclosed

in U. S. Patent No. 3,674,836, probucol and gemfibrozil being preferred, bile acid sequestrants such as cholestyramine, colestipol and DEAE-Sephadex (Secholex®, Polidexide®), as well as clofibrate, lipostabil (Rhone-Poulenc), Eisai E-5050 (an N-substituted ethanolamine derivative), imanixil (HOE-402) tetrahydrolipstatin (THL), istigmastanyl-phosphorylcholine (SPC, Roche), aminocyclodextrin (Tanabe Seiyoku), Ajinomoto AJ-814 (azulene derivative), melinamide (Sumitomo), Sandoz 58-035, American Cyanamid CL-277,082 and CL-283,546 (di-substituted urea derivatives), nicotinic acid, neomycin, p-aminosalicylic acid, aspirin, poly(diallylmethylamine) derivatives such as disclosed in U. S. Patent No. 4,759,923, quaternary amine poly(diallyldimethylammonium chloride) and ionenes such as disclosed in U. S. Patent No. 4,027,009, and other known serum cholesterol lowering agents which lower cholesterol through a mechanism other than by the inhibition of the enzyme HMG CoA reductase or squalene synthetase.

Also preferred are combinations of any of the HMG CoA reductase inhibitors or isoprenoid (phosphinylmethyl) phosphonates disclosed by Biller et al., supra with probucol or gemfibrozil.

In carrying out the method of the present invention, the cholesterol lowering drug may be administered to mammalian species, such as monkeys, dogs, cats, rats, humans, etc. and as such may be incorporated in a conventional systemic dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid of sodium bisulfite) or the like. Oral dosage forms are preferred, although parenteral forms are quite satisfactory as well.

The dose administered must be carefully adjusted according to age, weight and condition of the patient, as well as the route of administration, dosage form and regimen and the desired result.

Thus, for oral administration, a satisfactory result may be obtained employing the HMG CoA reductase inhibitor in dosages employed, for example, for lovastatin as indicated in the Physician's Desk Reference, such as in an amount within the range of from about 1 to 2000 mg, and preferably from about 4 to about 200 mg in. The squalene synthetase inhibitor may be employed in dosages in an amount within the range of from about 10 mg to about 2000 mg and preferably from about 25 mg to about 200 mg. The HMG CoA reductase inhibitor and squalene synthetase inhibitor may each be employed alone or may be employed together in the same oral dosage form or in separate oral dosage forms taken at the same time.

A preferred oral dosage form, such as tablets or capsules, will contain the HMG CoA reductase inhibitor in an amount of from about 0.1 to about 100 mg, preferably from about 5 to about 80 mg, and more preferably from about 10 to about 40 mg.

A preferred oral dosage form, such as tablets or capsules will contain the squalene synthetase inhibitor in an amount of from about 10 to about 500 mg, preferably from about 25 to about 200 mg.

The other serum cholesterol lowering agent when present will be employed in dosages normally employed as indicated in the Physician's Desk Reference, for each of such agents such as in an amount within the range of from about 2 mg to about 7500 mg and preferably from about 2 mg to about 4000 mg with the HMG CoA reductase inhibitor or squalene synthetase inhibitor and other serum cholesterol lowering agent being employed together in the same oral dosage form or in separate oral dosage forms taken at the same time.

The compositions described above may be administered in the dosage forms as described above in single or divided doses of one to four times daily. It may be advisable to start a patient on a low dose combination and work up gradually to a high dose combination.

Tablets of various sizes can be prepared, e.g., of about 2 to 2000 mg in total weight, containing one or both of the active substances in the ranges described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending one or the combination of active substances in a conventional liquid vehicle acceptable for pharmaceutical administration so as to provide the desired dosage in one to four teaspoonsful.

Such dosage forms can be administered to the patient on a regimen of one to four doses per day.

According to another modification, in order to more finely regulate the dosage schedule, the active substances may be administered separately in individual dosage units at the same time or carefully coordinated times. Since blood levels are built up and maintained by a regulated schedule of administration, the same result is achieved by the simultaneous presence of the two substances. The respective substances can be individually formulated in separate unit dosage forms in a manner similar to that described above.

Where combinations are desired, fixed combinations of HMG CoA reductase inhibitor and squalene synthetase inhibitor are and/or other cholesterol lowering agents are more convenient and are preferred,

especially in tablet or capsule form for oral administration.

In formulating the compositions, the active substances, in the amounts described above, are compounded according to accepted pharmaceutical practice with a physiologically acceptable vehicle, carrier, excipient, binder, preservative, stabilizer, flavor, etc., in the particular type of unit dosage form.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, aspartame, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

Some of the active substances described: above form commonly known, pharmaceutically acceptable salts such as alkali metal and other common basic salts or acid addition salts, etc. References to the base substances are therefore intended to include those common salts known to be substantially equivalent to the parent compound.

The formulations as described above will be administered for a prolonged period, that is, for as long as the potential for arteriosclerosis obliterans and intermittent claudication remains or the symptoms continue. Sustained release forms of such formulations which may provide such amounts biweekly, weekly, monthly and the like may also be employed. A dosing period of at least one to two weeks are required to achieve minimal benefit.

The following Examples represent preferred embodiments of the present invention. All temperatures are expressed in degrees Centigrade unless otherwise indicated and all mesh sizes are U.S. Standard ASTME.

## Example 1

A pravastatin formulation in the form of tablets having the following composition was prepared as described below.

| Ingredient | Parts by Weight |
|---|---|
| Pravastatin | 7 |
| Lactose | 67 |
| Microcrystalline cellulose | 20 |
| Croscarmellose sodium | 2 |
| Magnesium stearate | 1 |
| Magnesium oxide | 3 |

Pravastatin, magnesium oxide and a fraction (30%) of the lactose were mixed together for 2 to 10 minutes employing a suitable mixer. The resulting mixture was passed through a #12 to #40 mesh size screen. Microcrystalline cellulose, croscarmellose sodium and the remaining lactose were added and the mixture was mixed for 2 to 10 minutes. Thereafter, magnesium stearate was added and mixing was continued for 1 to 3 minutes.

The resulting homogeneous mixture was then compressed into tablets each containing 5 mg, 10 mg, 20 or 40 mg pravastatin which may be used to treat peripheral vascular disease (arteriosclerosis obliterans) and intermittent claudication.

## Example 2

Tablets of the following compositions are prepared as described below.

| Ingredient | Weight (mg) |
|---|---|
| (E,E)-[difluoro[hydroxy(4,8,12-trimethyl-3,7,11-tridecatrienyl)phosphinyl]methyl]phosphonic acid tripotassium salt (squalene synthetase inhibitor prepared as described by Biller et al. supra) | 100 mg |
| Avicel | 112.5 mg |
| Lactose | 113 mg |
| Cornstarch | 17.5 mg |
| Stearic Acid | 7 mg |
| | 350 mg |

The tablets are prepared from sufficient bulk quantities by slugging the squalene synthetase inhibitor Avicel, and a portion of the stearic acid. The slugs are ground and passed through a #2 screen and then mixed with the lactose, cornstarch, and the remainder of stearic acid. The mixture is compressed into 350 mg capsule shaped tablets in a tablet press. The tablets are scored for dividing in half.

The squalene synthetase inhibitor tablets may be administered as in accordance with the teachings of the present invention to treat arteriosclerosis obliterans and/or intermittent claudication. In addition, the pravastatin and squalene synthetase inhibitor tablets of Example 1 and 2 may be ground up into powders and used together in a single capsule.

## Example 3

A pravastatin formulation in the form of tablets, each containing 5 mg, 10 mg, 20 mg or 40 mg pravastatin, having the following composition was prepared as described in Example 1, except that color was incorporated into the powder mixture containing pravastatin, magnesium oxide and a fraction of the lactose.

| Ingredient | Parts by Weight |
|---|---|
| Pravastatin | 7 |
| Lactose | 67 |
| Microcrystalline cellulose | 20 |
| Croscarmellose sodium | 2 |
| Magnesium stearate | 1 |
| Magnesium oxide | 3 |
| FD&C Red #3 Lake | 0.2 |

The above pravastatin tablet may be administered to treat arteriosclerosis obliterans and/or intermittent claudication with or without a squalene synthetase inhibitor tablet (described in Example 2) in accordance with the teachings of the present invention.

## Examples 4 and 5

Lovastatin tablets are prepared employing conventional pharmaceutical techniques containing 20 mg lovastatin, cellulose, color, lactose, magnesium stearate and starch and butylated hydroxyanisole as a preservative as described in the 1988 PDR.

The lovastatin tablets may be employed alone or in combination with the squalene synthetase inhibitor tablets (described in Example 2) in separate or combined dosage forms to treat arteriosclerosis obliterans

or intermittent claudication in accordance with the present invention.

## Examples 6 to 8

A formulation in the form of tablets having the following composition is prepared as described in Example 1.

| Ingredient | Weight (mg) |
|---|---|
| (E,E,E)-[difluoro[hydroxy(4,8,12-trimethyl-1,3,7,11-tridecatetraenyl)phosphinyl]methyl]phosphonic acid tripotassium salt (squalene synthetase inhibitor prepared as described by Biller et al. supra) | 100 mg |
| Cornstarch | 50 mg |
| Gelatin | 7.5 mg |
| Avicel (microcrystalline cellulose) | 25 mg |
| Magnesium stearate | 2.5 mg |
| | 185 mg |

The above formulations alone or with pravastatin tablets, or lovastatin tablets described in Examples 1 and 4, respectively, or velostatin tablets may be employed in separate dosage forms or combined in a single capsule form to treat arteriosclerosis obliterans and/or intermittent claudication in accordance with the present invention.

## Example 9

Probucol tablets containing 250 mg probucol are prepared employing conventional procedures containing the following additional ingredients as set out in the 1988 PDR: corn starch, ethyl cellulose, glycerin, hydroxypropyl cellulose, hydroxypropylmethyl cellulose 2910, iron oxide, lactose, magnesium stearate, microcrystalline cellulose, polysorbate 80, talc and titanium dioxide.

The squalene synthetase inhibitor formulations or HMG CoA reductase inhibitor formulations described in the previous examples may be employed with probucol tablets as a combination in accordance with the teachings of the present invention to treat arteriosclerosis obliterans and/or intermittent claudication. In addition, any or all of the above drugs and probucol tablets may be ground up into powders and used together in a single capsule.

## Example 10

Capsules containing 300 mg gemfibrozil are prepared employing conventional pharmaceutical techniques containing the following additional ingredients as described in the 1988 PDR: polysorbate 80 NF, starch NF and silica gel.

The squalene synthetase inhibitor tablets or HMG CoA reductase inhibitor tablets previously described and gemfibrozil capsules may be administered as a combination or the HMG CoA reductase inhibitor or squalene synthetase inhibitor tablet may be ground into a powder and used in a single capsule containing gemfibrozil to treat arteriosclerosis obliterans and/or intermittent claudication.

## Examples 11

The above described HMG CoA reductase inhibitor tablets or squalene synthetase inhibitor tablets may be employed in combination with clofibrate capsules containing 500 mg clofibrate and inactive ingredients

including color, and gelatin as described in the 1988 PDR to treat arteriosclerosis obliterans and/or intermittent claudication in accordance with the present invention.

Examples 12

Squalene synthetase inhibitor tablets or HMG CoA reductase inhibitor tablets as described above may be employed in combination with cholestyramine resin containing 4 g cholestyramine, acacia, citric acid, color, flavor, polysorbate 80, propylene glycol alginate and sucrose as described in the 1988 PDR to treat arteriosclerosis obliterans and/or intermittent claudication in accordance with the present invention.

Examples 13

Squalene synthetase inhibitor tablets, or HMG CoA reductase inhibitor tablets described above may be employed in combination with nicotinic acid, colestipol, dextrothyroxine or other serum cholesterol lowering agent in accordance with the teaching of the present invention to treat arteriosclerosis obliterans and/or intermittent claudication.

It will also be appreciated that any of the HMG CoA reductase inhibitors or any of the squalene synthetase inhibitors disclosed herein may be employed in combination with each other and/or with any of the serum cholesterol lowering agents disclosed herein in accordance with the present invention.

**Claims**

1. Use of an inhibitor of the enzyme 3-hydroxy-3-methylglutaryl coenzyme A (HMG CoA) reductase or an inhibitor of the enzyme squalene synthetase or combinations thereof for the preparation of a pharmaceutical composition for treating arteriosclerosis obliterans and/or intermittent claudication in mammalian species.

2. Use according to Claim 1 wherein said inhibitor of the enzyme HMG CoA reductase is mevastatin, lovastatin, pravastatin or velostatin.

3. Use according to Claim 1 wherein said inhibitor of the enzyme HMG CoA reductase is a pyrazole analog of a mevalonolactone, an indene analog of mevalonolactone, a 3-carboxy-2-hydroxy-propane-phosphonic acid derivative, a 6-[2-(substituted-pyrrol-1-yl)-alkyl]pyran-2-one, an imidazole analog of mevalonolactone, or a heterocyclic analog of mevalonolactone, a naphthyl analog of mevalonolactone, an octahydro-naphthalene, fluindostatin, a keto analog of lovastatin or a 2,3-di-substituted pyrrole, furan or thiophene.

4. Use according to Claim 1 wherein the inhibitor of the enzyme squalene synthetase has the formula

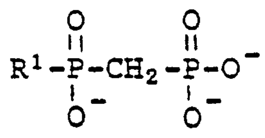 or 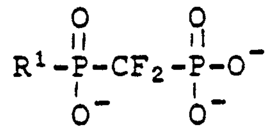

wherein $R^1$ is

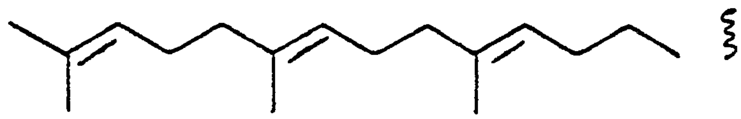,

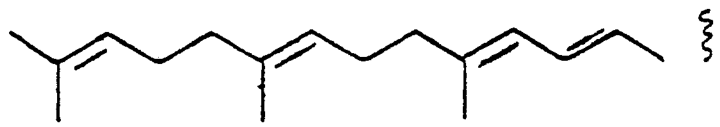,

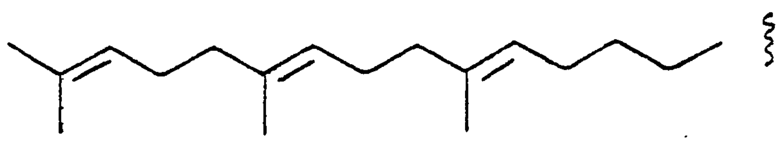, or

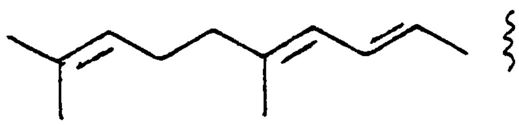.

5. Use according to Claim 1 or 2 wherein both the inhibitor of the enzyme HMG CoA reductase and the inhibitor of the enzyme squalene synthetase are present in a weight ratio of within the range of from about 0.001:1 to about 1000:1.

6. Use according to Claim 1 wherein the inhibitor of the enzyme squalene synthetase is employed alone.

7. Use according to Claims 1 or 2 wherein the inhibitor of the enzyme squalene synthetase or the HMG CoA reductase inhibitor is combined with a pharmaceutical which reduces serum cholesterol and/or inhibits cholesterol biosynthesis by a mechanism other than inhibiting production of the enzyme HMG CoA reductase or squalene synthetase.

8. Use according to Claim 7 wherein said pharmaceutical is probucol, gemfibrozil, clofibrate, dextrothyroxine or its sodium salt, colestipol or its hydrochloride, cholestyramine, nicotinic acid, neomycin, p-aminosalicylic acid or aspirin.

9. Use according to Claim 7 wherein said pharmaceutical is a bile acid sequestrant.

10. Use according to Claim 9 wherein said bile acid sequestrant is cholestyramine, colestipol, DEAE-Sephadex, a poly(diallylmethylamine) derivative, an ionene or the quaternary amine poly-(diallyldimethylammonium) chloride.

11. Use according to Claim 8 wherein said pharmaceutical is probucol or gemfibrozil.

12. Use according to Claim 7 wherein said pharmaceutical is probucol, gemfibrozil, nicotinic acid, cholestyramine, clofibrate, colestipol or p-aminosalicylic acid and the HMG CoA reductase inhibitor is pravastatin, lovastatin or velostatin.